# EUROPEAN PATENT APPLICATION

(11) **EP 1 900 776 A1**
(43) Date of publication of application: **19.03.2008**
(21) Application number: 06745653.3
(22) Date of filing: 25.04.2006
(51) Int. Cl.: C08L 71/02, C08G 65/32, A61L 27/00, C08J 3/24, C09K 3/00, C08B 37/16

(54) **GEL COMPOSITION AND METHOD FOR PRODUCING SAME**

(30) Priority: 25.04.2005 JP 2005126484
(71) Applicant: The University of Tokyo, Bunkyo-Ku, Tokyo 113-8654 (JP)
(72) Inventor: ITO, Kohzo c/o THE UNIVERSITY OF TOKYO, Tokyo 113-8654 (JP); KIDOWAKI, Masatoshi c/o THE UNIVERSITY OF TOKYO, Tokyo 113-8654 (JP); ZHAO, Changming c/o THE UNIVERSITY OF TOKYO, Tokyo 113-8654 (JP)
(74) Representative: HOFFMANN EITLE
(86) International application number: PCT/JP2006/308625
(87) International publication number: WO 2006/115255

(57) **Abstract**

A gel composition is provided, which in addition to being able to expect that various properties attributable to polyrotaxane will be retained, easily ensures stability, has superior shock absorbability and facilitates control of refractive index. The present invention provides a gel composition comprising a material having a network structure containing a polyrotaxane and a non-aqueous solvent, applications of the gel composition, and a process for preparing the gel composition.

## Description

### TECHNICAL FIELD

The present invention relates to a gel composition comprising a material having a network structure containing a polyrotaxane and a non-aqueous solvent, applications of the gel composition, and a process for preparing the gel composition.

### BACKGROUND ART

Polyrotaxanes have a linear molecule (axis) passing through openings of cyclic molecules (rotator) in a skewered manner so that the cyclic molecules include the linear molecule to form a pseudo-polyrotaxane in which blocking groups are arranged on both ends thereof (both ends of the linear molecule) to prevent elimination of the cyclic molecules. For example, research has recently been actively conducted on a polyrotaxane (see, for example, Patent Document 1), in which α-cyclodextrin (abbreviated as "CD") is used as a cyclic molecule and polyethylene glycol (abbreviated as "PEG") is used as a linear molecule, in consideration of its various properties.

Crosslinked polyrotaxane, in which corresponding polyrotaxanes have been crosslinked, not only makes it possible to control elasticity and viscoelasticity, but also enables safety to be easily secured by selecting PEG and CD, for example, for the raw materials, thereby leading to expectations of applications as a material for medical materials. Although crosslinked polyrotaxane is normally used in the form of a hydrogel (see, for example, Patent Document 2), problems attributable to water are therefore unable to be avoided. For example, when in the form of a hydrogel, the stability of the crosslinked polyrotaxane tends to be impaired since evaporation of water under the environment in which it is used cannot be completely prevented. In addition, although crosslinked polyrotaxane has elasticity, there are restrictions on its applications due to its inability to adequately absorb shocks. In addition, the development of, for example, a gel that facilitates control of refractive index, is expected in order to solve these problems as well as expand the range of applications thereof.
Patent Document 1: Japanese Patent No. 2810264
Patent Document 2: Japanese Patent No. 3475252

### DISCLOSURE OF THE INVENTION

### Problems to be solved by the Invention

An object of the present invention is to solve the aforementioned problems by providing a gel composition that can be expected to have various properties attributable to polyrotaxane while also facilitating the ensuring of stability. In addition, an object of the present invention is to provide a gel composition having superior shock absorbability as well as provide a gel composition that facilitates control of refractive index. Moreover, an object of the present invention is to provide applications for the gel composition and a process for preparing the gel composition.

### Means for Solving the Problems

The present invention relates to a gel composition comprising a material having a network structure containing a polyrotaxane and a non-aqueous solvent, applications of the gel composition, and a process for preparing the gel composition.

### Effects of the Invention

The gel composition of the present invention makes it possible to anticipate various properties attributable to polyrotaxane while also facilitating the ensuring of stability and being able to be applied to various products. In particular, the gel composition of the present invention has improved shock absorbability and facilitates control of refractive index, being able to, for example, make the refractive index of about 1.49 which is equal to that of polymethyl methacrylate (PMMA). In addition, in the case the gel composition of the present invention contains polyethylene glycol for the non-aqueous solvent, it is able to demonstrate moisture permeability equal to or greater than that of conventional silicone sheets (polydimethylsiloxane).

### BRIEF DESCRIPTION OF THE DRAWINGS

Fig. 1 shows changes in humidity in the case of a lower cell being in a saturated state and an upper cell being in a dry state.
Fig. 2 shows changes in humidity in the case of a lower cell being in a dry state and an upper cell being in a saturated state.

### BEST MODE FOR CARRYING OUT THE INVENTION

### Material Having Network Structure Containing Polyrotaxane

The gel composition of the present invention comprises a material having a network structure containing a polyrotaxane. Examples of such materials include a material containing in at least a portion thereof a structure in which corresponding polyrotaxanes are crosslinked, and a material containing in at least a portion thereof a structure in which a polyrotaxane and a polymer are crosslinked. Furthermore, an example of a structure in which cyclic molecules of polyrotaxanes are crosslinked by chemical bonds is the crosslinked polyrotaxane described in Japanese Patent No. 3475252.

### (Polyrotaxane or Polyrotaxane Molecule)

In the present description, a "polyrotaxane" or "polyrotaxane molecule" refers to a molecule having a linear molecule passing through openings of cyclic molecules in a skewered manner so that the cyclic molecules include the linear molecule to form a pseudo-polyrotaxane in which blocking groups are arranged on both ends thereof (both ends of the linear molecule) to prevent elimination of the cyclic molecules.

### (Linear Molecule)

In the present description, a linear molecule refers to a molecule or substance that is included by cyclic molecules and can be combined by non-covalent bonding. There are no particular limitations on these linear molecules provided they are linear, and any such molecules, including polymers, can be used.

The "linear chain" of the "linear molecule" refers to a substantially "linear chain". Namely, if a rotator in the form of a cyclic molecule is able to rotate or a cyclic molecule is able to slide or move over a linear molecule, then the linear molecule may have a branched chain. In addition, there are no particular limitations on the length of the "linear chain" provided it allows the cyclic molecules to slide or move over the linear molecule.

The "linear chain" of a "linear molecule" is determined relatively in the relationship with the polyrotaxane material. Namely, in the case of a material having a crosslinked structure in a portion thereof, the linear molecule may be present in only a very small portion of the material. However, even if present in only a very small portion, there are no particular limitations on the length thereof provided it allows the cyclic molecules to slide or move over the linear molecule.

Both hydrophilic and hydrophobic polymers can be used for the linear molecule. Examples of hydrophilic polymers include polyvinyl alcohol and polyvinyl pyrrolidone, poly(meth)acrylic acid, cellulose resins (such as carboxymethyl cellulose, hydroxyethyl cellulose or hydroxypropyl cellulose), polyacrylamide, polyethylene oxide, polyethylene glycol, polyvinyl acetal resins, polyvinyl methyl ether, polyamine, polyethylene imine, casein, gelatin, starch and/or copolymers thereof. Examples of hydrophobic polymers include polyolefin resins such as polyethylene, polypropylene and copolymer resins of other olefin monomers, polyester resins, polyvinyl chloride resins, polystyrene and polystyrene resins such as acrylonitrile-styrene copolymer resins, acrylic resins such as polymethyl methacrylate and (meth)acrylic acid ester copolymers, acrylonitrile- methyl acrylate copolymer resins, polycarbonate resins, polyurethane resins, vinyl chloride-vinyl acetate copolymer resins, polyvinyl butyral resins, and derivatives or modified forms thereof. Furthermore, polyisobutylene, polytetrahydrofuran, polyaniline, acrylonitrile-butadiene-styrene copolymer (ABS resin), polyamides such as Nylon, polyimides, polyisoprene, polydienes such as polybutadiene, polysiloxanes such as polydimethylsiloxane, polysulfones, polyimines, polyacetic anhydrides, polyureas, polysulfides, polyphosphazenes, polyketones, polyphenylenes, polyhaloolefins and derivatives thereof can be used.

Among these, polyethylene glycol, polyisoprene, polyisobutylene, polybutadiene, polypropylene glycol, polytetrahydrofuran, polydimethylsiloxane, polyethylene and polypropylene are preferable. Polyethylene glycol is particularly preferable.

The linear molecule itself preferably has a high fracture strength. Although the fracture strength of a compound or gel depends on the bond strength between the blocking groups and linear molecule, the bond strength between cyclic molecules and other factors as well, if the linear molecule itself has high fracture strength, higher fracture strength can be provided.

The number average molecular weight of the linear molecule is preferably 1,000 or more, and for example 1,000 to 1,000,000, more preferably 5,000 or more and for example 5,000 to 1,000,000, or 5,000 to 500,000, and even more preferably 10,000 or more and for example, 10,000 to 1,000,000, 10,000 to 500,000 or 10,000 to 300,000.

In addition, the linear molecule is preferably biodegradable molecule with respect to being "environmentally-friendly".

The linear molecule preferably has reactive groups on both ends thereof. As a result of having reactive groups, the linear molecule can easily react with the blocking groups. Although dependent upon the blocking groups used, examples of reactive group include a hydroxyl group, an amino group, a carboxyl group and a thiol group.

### (Cyclic Molecules)

In the present description, there are no particular limitations on the cyclic molecules provided they are able to include the aforementioned linear molecule, and any such cyclic molecules may be used.

"Cyclic molecules" refer to various cyclic substances including cyclic molecules. In addition, in the present invention, "cyclic molecules" refer to molecules or substances that are substantially cyclic. Namely, "substantially cyclic" includes molecules or substances that are not completely closed like in a letter "C" and may have a helical structure in which one end of the letter "C" overlaps the other end without being connected. Moreover, rings of "bicyclic molecules" to be described later can also be defined in the same manner as being "substantially cyclic" of "cyclic molecules". Namely, one or both rings of "bicyclic molecules" may be that which is not completely closed like in the letter "C", and may have a helical structure in which one end of the letter "C" overlaps the other end without being connected.

Examples of cyclic molecules include various cyclodextrins (such as α-cyclodextrin, β-cyclodextrin, γ-cyclodextrin, dimethyl cyclodextrin or glucosyl cyclodextrin and derivatives or modified forms thereof), crown ethers, benzo-crown ethers, dibenzo-crown ethers, dicyclohexano-crown ethers and derivatives or modified forms thereof.

The aforementioned cyclodextrins, crown ethers and the like have different sizes of the opening of the cyclic molecule depending on the type thereof. Thus, the cyclic molecule used can be selected according to the type of a linear molecule used, and more specifically, in the case of considering the linear molecule used to be cylindrical, according to the diameter of the cross-section of the cylinder, the hydrophobicity or hydrophilicity of the linear molecule and the like. In addition, in the case of using cyclic molecules having a relatively large opening and a cylindrical linear molecule having a relatively small diameter, two or more of the linear molecules can be included in the opening of the cyclic molecules.

Among these cyclic molecules, cyclodextrins are biodegradable, making them preferable with respect to being "environmentally-friendly".

α-Cyclodextrin is preferably used for the cyclic molecules while polyethylene glycol is preferably used for the linear molecule.

### (Blocking Groups)

There are no particular limitations on the blocking groups provided they are groups that allow the cyclic molecules to be maintained in a skewered state by the linear molecule, and any such groups may be used. Examples of such groups include groups having "bulkiness" and/or groups having "ionicity". Here, a "group" refers to various groups including molecular groups and polymer groups. Namely, a group having "bulkiness" may be group schematically represented in a spherical form, or a solid support represented in the manner of a sidewall. In addition, as a result of the mutual effects of the "ionicity" of a group having "ionicity" and the "ionicity" of a cyclic molecule, the cyclic molecule is able to be maintained in a skewered state by a linear molecule due to, for example, mutual repulsion.

In addition, the blocking group may be a polymer main chain or side chain provided it maintains a skewered state as described above. In the case the blocking group is a polymer A, the blocking group may be in a state in which polymer A serves as a matrix and a crosslinked structure is contained in a portion thereof, or it may be conversely be in a state in which a polyrotaxane material containing a crosslinked structure serves as the matrix and polymer A is contained in a portion thereof. In this manner, by combining with a polymer A having various properties, a composite material can be formed having a combination of the properties of a polyrotaxane material and the properties of polymer A.

More specifically, examples of blocking groups in the form of molecular groups include dinitrophenyl groups such as a 2,4-dinitrophenyl group or 3,5-dinitrophenyl group, cyclodextrins, adamantane groups, trityl groups, fluoresceins, pyrenes and derivatives or modified forms thereof. More specifically, even in the case of using α-cyclodextrin for the cyclic molecules and polyethylene glycol for the linear molecule, examples of blocking groups include cyclodextrins, nitrophenyl groups such as a 2,4-nitrophenyl group or 3,5-dinitrophenyl group, adamantane groups, trityl groups, fluoresceins, pyrenes and derivatives or modified forms thereof.

### (Crosslinked Polyrotaxane Structure)

A crosslinked polyrotaxane structure can be obtained by, for example, crosslinking polyrotaxane cyclic molecules by physical bonds and/or chemical bonds. Preferably two or more polyrotaxanes are used, in this case, they may be the same or different. Namely, a first polyrotaxane and a second polyrotaxane different from the first can be used. For example, although a first cyclic molecule contained a first polyrotaxane and a second cyclic molecule contained a second polyrotaxane can be crosslinked, at this time, the first cyclic molecule and the second cyclic molecule may be the same or different.

In the case of crosslinking by chemical bonds, the chemical bonds may be a direct bond or bond via various atoms or molecules. Examples of crosslinking by physical bonds include those using hydrogen bonding, Coulomb force, hydrophobic bonding, Van der Waals bonding and coordinate bonding. Furthermore, crosslinking includes that which reversibly changes from a non-crosslinked state or crosslinked state to a crosslinked state or non-crosslinked state depending on the presence or absence of an external stimulus. Namely, the case of reversibly changing from a non-crosslinked state to a crosslinked state due to a change in an external stimulus, and the opposite case, that is the case of reversibly changing from a crosslinked state to a non-crosslinked state due to a change in an external stimulus, are both included.

In the case of crosslinking cyclic molecules by chemical bonds, the cyclic molecules preferably having a reactive group on the outside of the ring. This is because this reaction group can be used to facilitate the reaction. Although the reactive group is dependent upon the crosslinking agent used and the like, examples of such groups include a hydroxyl group, amino group, carboxyl group, thiol group and aldehyde group. In addition, a group is preferably used that does not react with the blocking groups during the aforementioned blocking reaction.

Crosslinking is preferably that in which cyclic molecules are crosslinked using a crosslinking agent after having blocked both ends of pseudo-polyrotaxane. At this time, the conditions of the crosslinking reaction are generally conditions that prevent the blocking groups of the blocked polyrotaxane from being removed.

In addition, a first cyclic molecule can be crosslinked with a second cyclic molecule different therefrom. The first and second cyclic molecules can have reactive groups that are each capable of mutually reacting to form bonds.

### (Crosslinking Agent)

A crosslinking agent known in the prior art can be used for the crosslinking agent, examples of which include cyanuric chloride, trimethoyl chloride, terephthaloyl chloride, epichlorohydrin, dibromobenzene, glutaraldehyde, phenylene diisocyanate, tolylene diisocyanate (such as 2,4-tolylene diisocyanate), 1,1'-carbonyl diimidazole and divinylsulfone. Additional examples include various types of coupling agents such as silane coupling agents (such as various alkoxysilanes) and titanate coupling agents (such as various alkoxytitanes). Moreover, various types of photocrosslinking agents used for soft contact lens materials, including stilbazolium-based photocrosslinking agents such as formyl styrylpyridinium (see K. Ichimura et al., Journal of Polymer Science, Polymer Chemistry Edition, 20, 1411-1432 (1982), this document is incorporated herein for reference), and other photocrosslinking agents such as photocrosslinking agents functioning by photodimerization, and more specifically, cinnamic acid, anthracene and thymines, can also be used.

The molecular weight of the crosslinking agent is less than 2,000, preferably less than 1,000, more preferably less than 600 and most preferably less than 400.

In the case of using α-cyclodextrin for the cyclic molecules and crosslinking using a crosslinking agent, examples of crosslinking agents include cyanuric chloride, 2,4-tolylene diisocyanate, 1,1'-carbonyl diimidazole, trimethoyl chloride, terephthaloyl chloride and alkoxysilanes such as tetramethoxysilane or tetraethoxysilane. The use of α-cyclodextrin for the cyclic molecules and cyanuric chloride for the crosslinking agent is particularly preferable.

In the above description, a crosslinked structure was principally formed by crosslinking cyclic molecules after forming polyrotaxane. In addition thereto, a substance having a crosslinked cyclic molecular structure such as a "bicyclic molecule" having a first ring and a second ring can also be used. In this case, a crosslinked polyrotaxane of the present invention can be obtained by, for example, mixing "bicyclic molecule" with linear molecules, and including the linear molecule in the first ring and second ring of the "bicyclic molecules" in a skewered manner. In this case, both ends of the linear molecule are preferably blocked with blocking groups following inclusion.

### (Crosslinking Method)

A structure in which cyclic molecules of polyrotaxanes have crosslinked by chemical bonds can be prepared in the manner described below. First, cyclic molecules and linear molecules are mixed to prepare pseudo-polyrotaxane in which linear molecule is included in the openings of the cyclic molecules in a skewered manner. Various solvents may be used during the mixing of this preparation step. Examples of this solvent include solvents that dissolve cyclic molecules and/or linear molecules, and solvents that suspend the cyclic molecules and/or linear molecules. More specifically, the solvent can be suitably selected dependent upon the cyclic molecules and/or linear molecules used.

When preparing pseudo-polyrotaxane, it is preferable to control the amount of cyclic molecules that are skewered by the linear molecule. At least two cyclic molecules are preferably skewered by the linear molecule, so that at least two cyclic molecules include the linear molecule. In addition, in the case of defining the amount of cyclic molecules that can be maximally present on the linear molecule, or in other words the maximum inclusion amount, as 1, cyclic molecules are preferably present at a value of 0.001 to 0.6, preferably 0.01 to 0.5 and more preferably 0.05 to 0.4 the maximum inclusion amount.

The amount of cyclic molecules described above can be controlled according to the mixing time, temperature, pressure, increasing the molecular weight of the linear molecules used and the like. More specifically, for example, an excess of linear molecules may be dissolved in a saturated solution of cyclic molecules.

The pseudo-polyrotaxane is preferably such that cyclic molecules are not densely packed on the linear molecule as previously described. As a result of not being densely packed, the movable distance of crosslinked cyclic molecules or linear molecules can be maintained when crosslinked. A high fracture strength, high entropic elasticity, superior elasticity and/or superior recovery, as well as high absorbability or high hygroscopy as desired, can be provided depending on this movable distance as previously described. Next, polyrotaxane is prepared from the resulting pseudo-polyrotaxane by blocking both ends of the linear molecules with blocking groups to prevent elimination of cyclic molecules from the skewered state.

Two or more polyrotaxanes can be crosslinked by bonding the cyclic molecules of the resulting polyrotaxane by chemical bonds.

Next, an explanation is provided of a method for preparing a crosslinked structure in the case of using α-cyclodextrin for the cyclic molecules, polyethylene glycol for the linear molecule, 2,4-dinitrophenyl groups for the blocking groups, and cyanuric chloride for the crosslinking agent.

First, both ends of polyethylene glycol are modified with amino groups to obtain a polyethylene glycol derivative for the blocking treatment to be carried out later. The α-cyclodextrin and polyethylene glycol derivative are then mixed to prepare pseudo-polyrotaxane. During this preparation, in the case of defining the maximum inclusion amount to be 1, the mixing time can be made to be, for example, 1 to 48 hours, and the mixing temperature can be made to be, for example, 0 to 100°C so that the inclusion amount is 0.001 to 0.6 with respect to that value of 1.

In general, a linear molecule of polyethylene glycol having a number average molecular weight of 20,000 can be included a maximum of 230 α-cyclodextrins. Thus, this value is defined as the maximum inclusion amount. The aforementioned conditions allow an average of 60 to 65 (63) α-cyclodextrins to include a linear molecule of polyethylene glycol having a number average molecular weight of 20,000, namely a value of 0.26 to 0.29 (0.28) with respect to the maximum inclusion value, using polyethylene glycol. The inclusion amount of α-cyclodextrin can be confirmed by, for example, NMR, optical absorbance or elementary analysis.

The resulting pseudo-polyrotaxane is blocked by reacting it with 2,4-dinitrofluorobenzene dissolved in dimethylformamide (DMF) and thus polyrotaxane is obtained. Next, the resulting polyrotaxane is dissolved in aqueous sodium hydroxide solution. α-Cyclodextrin molecules are crosslinked by adding cyanuric chloride to this solution and allowing to react.

In addition, a crosslinked structure can also be obtained by the following method using crosslinked cyclic molecules, namely "bicyclic molecules", instead of the method described above. Namely, bicyclic molecules are first prepared. Bicyclic molecules have a first substantial ring and a second substantial ring as previously described. Next, a step is carried out in which the bicyclic molecules are mixed with first linear molecules and second linear molecules, and the first linear molecule is included in the openings of the first ring of the bicyclic molecules in a skewered manner and the second linear molecule is included in the openings of the second ring of the bicyclic molecules in a skewered manner to obtain a crosslinked structure composed of bicyclic molecules, followed by blocking both ends of the linear molecules to prevent elimination of the bicyclic molecules from the skewered state.

Furthermore, although the bicyclic molecules are indicated as being "bicyclic", they can also have one or two or more rings in addition to the first substantial ring and the second substantial ring. In addition, molecules may also be used as bicyclic molecules having a structure in which two of the letters "C" are bound to each other. In this case, the "C" shape can be opened after including the linear molecule in a skewered manner or after blocking with blocking groups. Furthermore, reference may be made to M. Asakawa et al., Angewante Chemie-International Edition 37(3), 333-337 (1998) and Asakawa et al., European Journal of Organic Chemistry 5, 985-994 (1999) with respect to molecules having a structure consisting of two bound C-shaped molecules and opening the rings of these molecules (these references are incorporated herein for references.

### (Introduction of Ionic and Nonionic Groups)

Ionic groups or nonionic groups can be introduced into moieties corresponding to cyclic molecules. The introduction of such groups makes it possible to change crosslinked density and form or affinity with a medium, or change properties such as swellability.

Ionic groups can be introduced by, for example, substituting at least a portion of cyclic molecules having hydroxyl groups (-OH) such as cyclodextrin with ionic groups.

There are no particular limitations on the ionic groups provided they have ionicity. Examples of ionic groups include -COOX group (wherein, X represents hydrogen (H), alkaline metal or other monovalent metal), -SO₃X group (wherein, X is defined as above), -NH₂ group, -NH₃X' group (wherein, X' represents a monovalent halogen ion), -PO₄ group and -HPO₄ group, and the ionic group is preferably at least one group selected from the group consisting of these groups.

It is preferred that groups having ionicity are substituted for 10 to 90%, preferably 20 to 80% and more preferably for 30 to 70% of all of the hydroxyl groups of all cyclic molecules.

The step for substituting a portion of the OH groups possessed by cyclic molecules with ionic groups may be carried out before, during or after the step for preparing pseudo-polyrotaxane. In addition, it may also be carried out before, during or after the step for preparing polyrotaxane by blocking the pseudo-polyrotaxane. Moreover, it may also be carried out before, during or after the step for crosslinking polyrotaxanes. In addition, it can also be provided at two or more of these times. The substitution step is preferably carried out after preparing polyrotaxane by blocking pseudo-polyrotaxane, but before crosslinking the polyrotaxanes. Although depending on the ionic groups used for substitution, there are no particular limitations on the conditions used in the substitution step, and various reaction methods and reaction conditions can be used. For example, in the case of using one of the types of the aforementioned groups in the form of a carboxyl group for the ionic group, examples of substitution methods include, but are not limited to, oxidation of primary hydroxyl groups, conversion of primary and secondary hydroxyl groups to ether derivatives (including carboxymethylation and carboxyethylation), and addition of succinic anhydride, maleic anhydride and/or a derivative thereof.

On the other hand, introduction of nonionic groups can be carried out by, for example, substituting at least a portion of cyclic molecules having hydroxyl groups (-OH) such as cyclodextrin with nonionic groups.

Nonionic groups preferably have an -OR group. Here, R preferably represents a linear or branched alkyl group having 1 to 12 carbon atoms, a linear or branched alkyl group having 2 to 12 carbon atoms and containing at least one ether group, a cycloalkyl group having 3 to 12 carbon atoms, a cycloalkyl ether group having 2 to 12 carbon atoms, or a cycloalkyl thioether group having 2 to 12 carbon atoms. Furthermore, examples of R include, but are not limited to, linear alkyl groups such as methyl, ethyl, propyl, butyl, pentyl, hexyl, heptyl, octyl, nonyl, decyl, undecyl and dodecyl groups; branched alkyl groups such as isopropyl, isobutyl, tert-butyl, 1-methylpropyl, isoamyl, neopentyl, 1,1-dimethylpropyl, 4-methylpentyl, 2-methylbutyl and 2-ethylhexyl groups; cycloalkyl groups such as cyclopropyl, cyclobutyl, cyclopentyl, cyclohexyl, cycloheptyl, cyclooctyl and adamantyl groups; cycloalkyl ether groups such as ethylene oxide, oxetane, tetrahydrofuran, tetrahydropyran, oxepane, dioxane and dioxolane groups; and, cycloalkyl thioether groups such as thiirane, thietane, tetrahydrothiophene, thiane, dithiolane and dithiane. Among these, R is preferably a methyl, ethyl, propyl, butyl, pentyl or hexyl group, and more preferably a methyl, ethyl or propyl group.

In addition, the nonionic group is preferably an -OR'-X group. Here, R' refers to groups in which a hydrogen has been removed from the aforementioned R, and X is preferably OH, NH₂ or SH. Furthermore, R' defined independently of R. In addition, preferable examples of R' include groups in which a single hydrogen has been removed from a methyl, ethyl, propyl, butyl, pentyl or hexyl group, while preferable examples include groups in which a single hydrogen has been removed from a methyl, ethyl or propyl group. X is preferably OH or NH₂ and more preferably OH.

Moreover, nonionic groups are preferably an -O-CO-NH-R₁ group, -O-CO-R₂ group, O-Si-R₃ group or -O-CO-O-R₄ group. Preferably, R₁, R₂, R₃ and R₄ independently represent a linear or branched alkyl group having 1 to 12 carbon atoms, a linear or branched alkyl group having 2 to 12 carbon atoms and containing at least one ether group, a cycloalkyl group having 3 to 12 carbon atoms, a cycloalkyl ether group having 2 to 12 carbon atoms or a cycloalkyl thioether group having 2 to 12 carbon atoms.

It is preferred that nonionic groups are substituted for 10 to 90%, preferably 20 to 80% and more preferably for 30 to 70% of all of the hydroxyl groups of all cyclic molecules.

The step for substituting hydroxyl groups possessed by cyclic molecules with nonionic groups may be carried out before, during or after the step for preparing pseudo-polyrotaxane. In addition, it may also be carried out before, during or after the step for preparing polyrotaxane by blocking pseudo-polyrotaxane. Moreover, it can also be carried out before, during or after the step for crosslinking the polyrotaxanes. This step can also be provided at two or more times. The substitution step is preferably carried out after preparing polyrotaxane by blocking pseudo-polyrotaxane, but before crosslinking the polyrotaxanes. Although depending on the nonionic groups used for substitution, there are no particular limitations on the conditions used in the substitution step, and various reaction methods and reaction conditions can be used. For example, in the case of using the aforementioned -OR group as a nonionic group, namely in the case of forming ether bonds, the following method can be used. In general, a method is used in which a halide is present using a suitable base in a polar solvent such as dimethylsulfoxide or dimethylformamide. Examples of bases that can be used include alkaline metal salts or alkaline earth metal salts such as sodium methoxide, sodium ethoxide, potassium t-butoxide, sodium hydroxide, potassium hydroxide, cesium hydroxide, lithium hydroxide, potassium carbonate, cesium carbonate, barium hydroxide, barium oxide, sodium hydride or potassium hydride. Silver oxide can also be used. In addition, other examples include a method in which a leaving group such as a p-toluenesulfonyl group or methanesulfonyl group is introduced followed by substituting with a suitable alcohol.

Further, other examples of methods in addition to the method for introducing a nonionic group in the form of an
- OR group by ether bonding as described above include a method using the carbamate bond formation by an isocyanate compound and the like, a method using ester bond formation by a carboxylic acid compound, acid chloride compound or acid anhydride, a method using silyl ether bond formation by a silane compound, and a method using carbonate bond formation by a chlorocarbonic acid compound.

Furthermore, an ionic group and nonionic group can be introduced into cyclic molecules via a compound containing two or more reactive groups. Examples of compounds having two or more reactive groups include the aforementioned crosslinking agents, and more specifically, cyanuric chloride, ethylene glycol glycidyl ether, glutaraldehyde and derivatives thereof. In these cases, crosslinking groups that contribute to crosslinking function as reactive groups. Namely, an ionic group and nonionic group can be introduced as a result of a portion of the reactive groups (crosslinking groups) contained in these compounds bonding to the cyclic molecules, and another portion of the reactive groups (crosslinking groups) bonding to ionic group-containing compounds or nonionic group-containing compounds. This state can be represented by, for example, the following formula I. Here, L represents a single bond or a monovalent group that bonds with a cyclic molecule, and one or both of X and Y represent a group having an ionic group or nonionic group. In the case one of X and Y is an ionic group or nonionic group, the other may be bonded with a cyclic molecule. In this case, although the ionic group or nonionic group is dependent upon the crosslinking portion, introduction of an ionic group or nonionic group into a cyclic molecule includes this form of introduction.

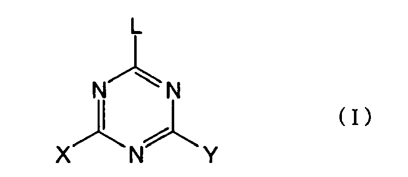

There are no particular limitations on the ionic group-containing compound provided it has the property of reacting with a crosslinking agent and has an ionic group following reaction, examples of which include compounds having two or more functional groups such as amino acids and derivatives thereof. In addition, there are no particular limitations on the nonionic group-containing compound provided it has the property of reacting with a crosslinking agent and has a nonionic group following reaction.

Although introduction of ionic groups or nonionic groups may be carried out before, during or after crosslinking polyrotaxanes, it is preferably carried out after crosslinking. Although dependent upon the groups used in the reaction, there are no particular limitations on the reaction conditions, and various reaction methods and reaction conditions can be used, examples of which include, but are not limited to, acid chloride reactions and silane coupling reactions.

### Structure Having Crosslinked Polyrotaxane and Polymer

In addition, a structure in which polyrotaxane and a polymer are crosslinked can be obtained by crosslinking the cyclic molecules of polyrotaxane with a polymer by chemical bonds and/or physical bonds. In the case of crosslinking by chemical bonds, the chemical bonds may be a direct bond or bond via various atoms or molecules. Examples of crosslinking by physical bonds include those using hydrogen bonding, Coulomb force, hydrophobic bonding, Van der Waals bonding and coordinate bonding. Furthermore, crosslinking includes that which reversibly changes from a non-crosslinked state or crosslinked state to a crosslinked state or non-crosslinked state depending on the presence or absence of an external stimulus. Namely, the case of reversibly changing from a non-crosslinked state to a crosslinked state due to a change in an external stimulus, and the opposite case, that is the case of reversibly changing from a crosslinked state to a non-crosslinked state due to a change in an external stimulus, are both included.

Cyclic molecules of polyrotaxane and polymer are preferably chemically bonded by a crosslinking agent. The cyclic molecules preferably have at least one type of group selected from the group consisting of an -OH group, -NH₂ group, -COOH group, epoxy group, vinyl group, thiol group and photocrosslinkable group. This is because these groups can be reacted to carry out crosslinking. Furthermore, examples of photocrosslinkable groups include, but are not limited to, cinnamic acid, coumarin, chalcone, anthracene, styrylpyridine, styrylpyridinium salt and styrylquinolium salt. The previously described specific examples and preferable examples of crosslinking agents are applied for the crosslinking agent.

### (Polymer)

Although there are no particular limitations on the polymer, it preferably has at least one group selected from the group consisting of an -OH group, -NH₂ group, -COOH group, epoxy group, vinyl group, thiol group and photocrosslinkable group in the main chain or a side chain thereof. This is because these groups can be reacted to carry out crosslinking. Furthermore, examples of photocrosslinkable groups include, but are not limited to, cinnamic acid, coumarin, chalcone, anthracene, styrylpyridine, styrylpyridinium salt and styrylquinolium salt.

The polymer may be a homopolymer or copolymer. Two or more types of polymers may be used, and in the case of using two more types of polymers, at least one type of polymer is preferably bonded with polyrotaxane via cyclic molecules. In the case a polymer used as a material of the present invention is a copolymer, it may comprise two, three of more kinds of monomer. As to the case of a copolymer it is preferably a block copolymer, alternating copolymer, random copolymer or graft copolymer and the like. The number average molecular weight of the polymer is preferably 1,000 to 1,000,000 and more preferably 10,000 to several hundred thousand.

Examples polymers include, but are not limited to, polyvinyl alcohol, polyvinyl pyrrolidone, poly(meth)acrylic acid, cellulose-based resin (such as carboxymethyl cellulose, hydroxyethyl cellulose and hydroxypropyl cellulose), polyacrylamide, polyethylene oxide, polyethylene glycol, polypropylene glycol, polyvinyl acetal-based resin, polyvinyl methyl ether, polyamine, polyethylene amine, casein, gelatin, starch and/or copolymers thereof, polyolefin-based resin such as polyethylene, polypropylene, copolymer resins of other olefin-based monomers, polyester resin, polyvinyl chloride resin, polystyrene-based resin such as polystyrene, acrylonitrile-styrene copolymer resin, acrylic-based resin such as polymethyl methacrylate and (meth)acrylic acid ester copolymers, acrylonitrile-methyl acrylate copolymer resin, polycarbonate resin, polyurethane resin, vinyl chloride-vinyl acetate copolymer resin, polyvinyl butyral resin and derivatives or modified forms thereof, polyisobutylene, polytetrahydrofuran, polyaniline, acrylonitrile-butadiene-styrene copolymer resin (ABS resin), polyamides such as Nylon, polyimides, polyisoprene, polydienes such as polybutadiene, polysiloxanes such as polydimethylsiloxane, polysulfones, polyimines, polyacetic anhydrides, polyureas, polysulfides, polyphosphazenes, polyketones, polyphenylenes, polyhaloolefins and derivatives and modified forms thereof. Furthermore, said derivatives are preferably those have at least one group selected from the group consisting the aforementioned groups, namely an -OH group, -NH₂ group, -COOH group, epoxy group, vinyl group, thiol group and photocrosslinkable group.

### (Crosslinking Method)

A structure in which cyclic molecules of polyrotaxane and polymer are crosslinked by chemical bonds can be prepared in the manner described below. Namely, this structure can be prepared by a method comprising: a) a step for mixing polyrotaxane (refer to the aforementioned description regarding the preparation thereof) and polymer; b) optionally, a step for crosslinking at least a portion of the polymer by physical bonds and/or chemical bonds; and c) a step for crosslinking at least a portion of the polymer and at least a portion of the cyclic molecules of polyrotaxane by physical bonds and/or chemical bonds.

In step a), the weight ratio of polyrotaxane and polymer (polyrotaxane/polymer) is preferably 1/1000 or more, more preferably 1/500 or more and even more preferably 1/100 or more from a viewpoint of demonstrating various properties attributable to polyrotaxane. However, the weight ratio thereof is not limited to these ranges, but rather the amount of polymer used can be increased to a weight ratio of 1/1000 to 1/2 or the amount of polyrotaxane used can be increased to a weight ratio of 1/2 to 1000 according to the desired properties.

In step b), at least a portion of the polymer is preferably chemically crosslinked. Chemical crosslinking can be carried out using, for example, a crosslinking agent. Examples of crosslinking agents include, but are not limited to, those listed previously.

The aforementioned step c) may be carried out before or after step b). In addition, step b) and step c) may also be carried out nearly simultaneously.

The mixing step of step a) may be carried out in the absence or presence of a solvent depending on the polymer used. In the case of using a solvent, examples of solvents include, but are not limited to, water, toluene, xylene, benzene, anisole, cyclohexanone, N-methylpyrrolidone, dimethylformamide, dimethylacetoamide, methyl ethyl ketone, chloroform, dichloromethane, carbon tetrachloride, hexafluoroisopropyl alcohol, tetrahydrofuran, dioxane, acetone, ethyl acetate, dimethylsulfoxide and acetonitrile.

Step b) is preferably carried out under conventionally known polymer crosslinking conditions. Examples of conditions include, but are not limited to, the examples indicated below. For example, i) in the case the polymer has an active substituent such as an epoxy group, the crosslinking reaction can be carried out in the presence of heat or active hydrogen in the manner of an amine or acid anhydride. In addition, the crosslinking reaction can also be carried out by irradiating with light in the presence of a photo acid generator or photo base generator. ii) In the case the polymer has an unsaturated double bond such as a vinyl group, the crosslinking reaction can be carried out by heating or irradiating with light in the presence of heat or a photo radical generator. iii) In the case the polymer has a photocrosslinkable group as described above, the crosslinking reaction can be carried out by heating or irradiating with light. iv) In the case the polymer has a hydroxyl group, amino group or carboxyl group and the like, the crosslinking reaction can be carried out in the presence of a poly-substituted isocyanate, carbodiimide, triazine or silane. v) Even in the case the polymer does not have various groups, the crosslinking reaction can still be carried out by irradiation with an electron beam.

In step c), crosslinking is preferably carried out by chemical bonds. Crosslinking is preferably carried out by chemically reacting a group on the main chain and/or side chain of the polymer such as an -OH group, -NH₂ group, -COOH group, epoxy group, vinyl group, thiol group or photocrosslinkable group, with a group possessed by the cyclic molecules such as an -OH group, -NH₂ group, -COOH group, epoxy group, vinyl group, thiol group or photocrosslinkable group. The conditions for step c) depend on the group possessed by the polymer, the group possessed by the cyclic molecules and the like. The crosslinking conditions described above, can be similarly used for the conditions of step c), but are not limited thereto.

In addition, a structure in which cyclic molecules of polyrotaxane and a polymer are crosslinked by chemical bonds can also be prepared in the manner described below. Namely, this structure can be prepared by a method comprising: a) a step for mixing polyrotaxane with a monomer that composes a polymer; b) a step for forming a polymer by polymerizing the monomer; c) optionally, a step for crosslinking at least a portion of the polymer by physical bonds and/or chemical bonds; and d) a step for crosslinking at least a portion of the polymer and at least a portion of the cyclic molecules of polyrotaxane by chemical bonds.

In step a), the weight ratio of the polyrotaxane and monomer (polyrotaxane/monomer) is preferably 1/1000 or more, more preferably 1/500 or more and even more preferably 1/100 or more from a viewpoint of demonstrating various properties attributable to polyrotaxane. However, the weight ratio is not limited thereto, but rather, for example, the amount of polymer used can be increased to a weight ratio of 1/1000 to 1/2 or the amount of polyrotaxane used can be increased to a weight ratio of 1/2 to 1000 according to the desired properties.

In step c) of the aforementioned method, at least a portion of the polymer is preferably chemically crosslinked. Chemical crosslinking can be carried out using, for example, a crosslinking agent. Examples of crosslinking agents include, but are not limited to, those listed previously.

In the aforementioned method, step b) and step c) are preferably carried out nearly simultaneously. In addition, step c) and step d) are also preferably carried out nearly simultaneously. Moreover, step b), step c) and step d) may also be carried out nearly simultaneously. In addition, step d) may be carried out before or after step c).

The conditions of the step for forming the polymer by polymerizing the monomer depend on the monomer used and the like. Conventionally known conditions can be used for the conditions thereof.

Furthermore, an ionic group or nonionic group can be introduced into the cyclic molecules of polyrotaxane as previously described.

### Medium Containing Non-Aqueous Solvent

The gel composition of the present invention contains a non-aqueous solvent as a medium. In the present description, a non-aqueous solvent refers to a liquid other than water and may be a single liquid or a mixture of a plurality of liquids. In general, gelling ability changes according to the relationship between the medium and network structure, and it may be difficult to form a gel depending on the combination thereof. However, a network structure containing a polyrotaxane as in the present invention is able to retain various media, a gel composition having desired properties is obtained according to selection of the medium. In addition, stability is also easily ensured as a result of containing a non-aqueous solvent in the medium.

Examples of non-aqueous solvents that can be used in the gel composition of the present invention natural oils such as glycerin, castor oil and olive oil; polyvalent alcohols such as ethylene glycol, diethylene glycol, triethylene glycol, tetraethylene glycol, propylene glycol, dipropylene glycol, 1,3-butanediol, 1,4-butanediol, hexylene glycol, octylene glycol, polyethylene glycol, polypropylene glycol and polyester polyol; fatty acids and particularly higher fatty acids such as oleic acid and linolenic acid; ethers such as alkyl ethers of polyvalent alcohols and ethylene oxide-propylene oxide copolymers; ethyl abietate; and, silicone oils such as dimethyl silicone oil, methyl phenyl silicone oil and methyl hydrogen silicon oil. Alkyl ethers of polyvalent alcohols may be monoalkyl ethers or polyalkyl ethers, examples of which include lower alkyl ethers of polyvalent alcohols having 1 to 6 carbon atoms such as monomethyl ethers, dimethyl ethers, monoethyl ethers and diethyl ethers of polyvalent alcohols. Polyethylene glycol preferably has a number average molecular weight of 200 to 600 and more preferably 200 to 450, while polypropylene glycol preferably has a number average molecular weight of 400 to 5000 and more preferably 400 to 3500, although not limited thereto. In addition, esters of polyvalent alcohols can also be used, examples of which include (meth)acrylic acid esters (such as ethylene glycol mono(meth)acrylate, polyethylene glycol mono(meth)acrylate, polypropylene glycol mono(meth)acrylate, ethylene glycol di(meth)acrylate, polyethylene glycol di(meth)acrylate and polypropylene glycol di(meth)acrylate. Branched polyethylene glycol, branched polypropylene glycol and esters thereof (such as (meth)acrylic acid esters) can also be used, examples of which include (meth)acrylic acid esters of branched polyethylene glycol. Moreover, various modified silicone oils can also be used, examples of which include (meth)acrylic acid-modified silicone oil, and particularly silicon oils in which the ends thereof are modified with (meth)acrylic acid.

Non-aqueous solvents that are liquids at room temperature are preferable from a viewpoint of handling and the like. However, Non-aqueous solvents that are in liquid form at a temperature above room temperature (such as 50 to 200°C) can also be used depending on the application.

Liquids having a viscosity greater than 1 cP (20°C) are particularly preferable from a viewpoint of shock absorbability. Examples of such liquids include the aforementioned natural oils, polyvalent alcohols, fatty acids, polyethers and ethyl abietate, and in the case of silicone oil, that having a degree of polymerization of 3 or more is preferable. These liquids more preferably have a viscosity of 5 cP (20°C) or more and even more preferably a viscosity of 10 cP (20°C) or more. Although highly fluid liquids having a viscosity of, for example, 5 to 1000 cP (20°C) and particularly 5 to 500 cP (20°C) can be used from a viewpoint of handling ease during gelling, the viscosity thereof is not limited to these ranges, but rather liquids having a viscosity of several ten thousand cP (20°C), such as a viscosity of 10,000 cP (20°C), can also be used.

In addition, the gel composition of the present invention allows the refractive index to be changed depending on the non-aqueous solvent selected. Since the refractive index of the medium is essentially the same as the refractive index of the gel composition, a non-aqueous medium having a high refractive index could be selected to increase the refractive index of the gel composition. Since the refractive index of water at the Na-D line and 20°C (refractive index indicates that at the Na-D line and 20°C unless specifically indicated otherwise) is 1.33, in order to obtain a higher refractive index, a non-aqueous solvent having a refractive index of, for example, 1.37 to 1.60, can be used. Examples of such non-aqueous solvents include silicone oil and polyvalent alcohol. In particular, polyethylene glycol and polypropylene glycol have refractive indices on the order of 1.44 to 1.46, and are particularly suitable for the gel composition of the present invention as alternatives to the polymethyl methacrylate (refractive index: 1.49).

Furthermore, the medium can also contain optional components such as cationic surfactant, anionic surfactant, nonionic surfactant, antioxidant, heat stabilizer, ultraviolet absorber, disinfectant, pigment, colorant or fragrance. In addition, water can also be contained within a range that does not impair the object of the present invention. This includes water that inevitably enters the non-aqueous solvent or gel composition production process due to moisture absorption and the like.

Although dependent upon the type of raw materials of the polyrotaxane material, the degree of expansion of the gel composition of the present invention is greater than 1 (weight of gel composition/dry weight of polyrotaxane material) based on the dry weight of the polyrotaxane material, and can be, for example, 1.1 to 1000 times the dry weight of the polyrotaxane material. For example, that having a degree of expansion of about 1.1 times to about 100 times can be used in applications requiring hardness such as artificial cartilage.

### Process for Preparing Gel Composition

The gel composition of the present invention can be prepared by immersing a polyrotaxane material containing a crosslinked structure in a medium containing a non-aqueous solvent and other optional components. The polyrotaxane material may be in a dry state or in a state of containing a solvent in the case of having been crosslinked in a solvent, and may be immersed in a desired medium. However, in the latter method, the solvent is water and the desired medium is not soluble in water, or in the case of a non-aqueous solvent having low solubility, the polyrotaxane material is first immersed in an intermediate solvent in which both water and the desired medium dissolve to replace the water with the intermediate solvent, and then immersing in the desired medium.

In addition, the gel composition of the present invention can be produced by carrying out crosslinking of polyrotaxanes or crosslinking of polyrotaxane and polymer in a medium containing a non-aqueous solvent and other optional components.

Thus, the gel composition of the present invention uses a material having a network structure containing a polyrotaxane, which in addition to making it possible to anticipate various properties attributable to polyrotaxane, facilitates ensuring of stability as a result of containing a non-aqueous solvent as well as it enabling it to be applied to various products. In particular, shock absorbability can be improved depending on the non-aqueous solvent selected for use in the present invention. In addition, the non-aqueous solvent of the present invention facilitates control of refractive index, thereby enabling it to have a refractive index of, for example, about 1.49 which is equal to that of polymethyl methacrylate.

A typical example of a non-aqueous solvent used in the gel composition of the present invention is polyethylene glycol. In particular, that having a number average molecular weight of 200 to 600 is preferable, while that having a number average molecular weight of 200 to 450 is more preferable from a viewpoint of ease of handling. In addition, the medium is substantially composed of polyethylene glycol.

The viscosity of this polyethylene glycol is greater than 1 cP (20°C), and contributes to improvement of shock absorbability. For example, the crosslinked structure of the polyrotaxane material of the gel composition of the present invention can be expected to have greater shock absorbability in terms of the 0.1 Hz vibration absorption coefficient (Tanδ) by a factor of ten as compared with a similar hydrogel.

Moreover, as was previously described, the refractive index of polyethylene glycol is about 1.46, and the refractive index of the gel composition can be made to be at the level of about 1.49 equal to the refractive index of polymethyl methacrylate (PMMA) commonly used as a clear plastic. Consequently, the gel composition of the present invention is useful as an ophthalmic device such as a contact lens material.

In addition, a gel composition using polyethylene glycol can be expected to demonstrate performance equal to or better than that of silicone sheets considered to be materials having superior moisture permeability. Since conventional hydrogels naturally contain water, they are unable to be evaluated on the basis of moisture permeability.

In particular, the combination of α-CD for the cyclic molecules and polyethylene glycol for the linear molecule (preferably having a number average molecular weight of 10,000 to 1,000,000, more preferably 10,000 to 500,000 and even more preferably 10,000 to 300,000) is preferable. This is because this combination makes it possible to expect the obtaining of a gel composition in a homogeneous state due to the favorable affinity thereof. There are no particular limitations on the blocking groups and crosslinking agent provided they are those of which examples were previously listed, and ionic groups or nonionic groups may be introduced.

Although polyrotaxane where a linear molecule such as polyethylene glycol and the like is included in cyclic molecules of α-CD is typically insoluble in water, it is soluble in aqueous alkaline solution. Consequently, in the case of crosslinking polyrotaxanes, crosslinking is frequently carried out by dissolving the polyrotaxane in such an aqueous solution. Optionally, the alkaline aqueous solution is replaced with pure water or saline followed by replacing with a medium containing a non-aqueous solvent. As previously described, the gel composition of the present invention can contain water within a range that does not impair the object thereof, and water entering from the production process is included therein.

Furthermore, an alkyl ether of the aforementioned polyethylene glycol can also be used preferably, examples of which include mono-lower alkyl ethers and di-lower alkyl ethers such as polyethylene glycol monomethyl ether, polyethylene glycol dimethyl ether and polyethylene glycol diethyl ether.

Although the following provides a more detailed explanation of the present invention based on examples thereof, the present invention is not limited to these

### examples.

### Preparation Example 1: Polyrotaxane

Polyrotaxane of Production Example 1 was prepared in the manner described below.

### <Preparation of PEG-Carboxylic Acid by TEMPO Oxidation of PEG>

10 g of PEG (number average molecular weight: 35,000), 100 mg of TEMPO (2,2,6,6-tetramethyl-1-piperidinyloxy radical) and 1 g of sodium bromide were dissolved in 100 ml of water. 5 ml of a commercially available aqueous sodium hypochlorite solution (effective chlorine concentration: about 5%) were added to the resulting solution and allowed to react while stirring at room temperature. Although the pH of the system decreased rapidly immediately after addition as the reaction progressed, 1 N NaOH was added to adjust the pH so as to maintain as close to pH 10 to 11 as possible. Although the decrease in pH was no longer observed after about 3 minutes, stirring was continued for an additional 10 minutes. The reaction was terminated by adding ethanol within a range of up to a maximum of 5 ml. Extraction with 50 ml of methylene chloride was repeated 3 times and after components other than inorganic salt had been extracted, the methylene chloride was distilled off with an evaporator. After dissolving in 250 ml of warm ethanol, the solution was placed in a refrigerator at -4°C overnight to precipitate PEG-carboxylic acid, namely PEG having carboxylic acid (-COOH) substituted on both ends thereof. The precipitated PEG-carboxylic acid was recovered by centrifugal separation. Several cycles of this dissolving in warm ethanol, precipitation and centrifugation were repeated followed finally by drying by vacuum drying to obtain PEG-carboxylic acid. The yield was 95% or more. The carboxylation ratio was 95% or more.

### <Preparation of Inclusion Complex Using PEG-Carboxylic Acid and α-CD>

After dissolving 3 g of the PEG-carboxylic acid prepared above and 12 g of α-CD in separately prepared 50 ml aliquots of warm water at 70°C, both solutions were mixed followed by allowing to stand undisturbed overnight in a refrigerator (4°C). The inclusion complex that precipitated in the form of a cream was freeze-dried and recovered. The yield was 90% or more (about 14 g).

### <Blocking of Inclusion Complex Using Adamantane Amine and BOP Reagent Reaction System>

0.13 g of adamantane amine were dissolved in 50 ml of dimethylformamide (DMF) at room temperature followed by addition of 14 of the inclusion complex obtained above and promptly mixing by shaking well. Continuing, this was then added to a solution of 0.38 g of BOP reagent (benzotriazol-1- yl-oxy-tris(dimethylamino)phosphonium hexafluorophosphate) dissolved in 25 ml of DMF followed by similarly mixing by shaking. Moreover, this was then added to a solution of 0.14 ml of diisopropyl ethyl amine in 25 ml of DMF followed by similarly mixing by shaking. The resulting mixture was allowed to stand undisturbed overnight in a refrigerator (4°C). Subsequently, 100 ml of 1:1 DMF/methanol mixed solution were added and mixed well followed by centrifugal separation and discarding the supernatant. After repeating washing with DMF/methanol mixed solution twice, washing using 100 ml of methanol was further repeated twice by centrifugal separation in the same manner. After vacuum-drying the resulting precipitate, the dried precipitate was dissolved in 50 ml of dimethylsulfoxide (DMSO), and the resulting clear solution was dropped into 700 ml of water to precipitate polyrotaxane. The precipitated polyrotaxane was recovered by centrifugal separation and either vacuum-dried or freeze-dried. This cycle of dissolving in DMSO, precipitating in water, recovery and drying was repeated twice to finally obtain purified polyrotaxane. The yield based on the added inclusion complex was about 68% (9.6 g from 14 g of inclusion complex).

### Preparation Example 2: Methylated Polyrotaxane

Hydroxyl groups of cyclic molecules of the polyrotaxane of Preparation Example 1 were methylated to obtain methylated polyrotaxane of Preparation Example 2. The following provides a detailed description thereof.
1.0 g of the polyrotaxane of Preparation Example 1 was dissolved in 10 ml of dehydrated DMSO followed by the addition of 1.7 g of sodium methoxide (28% methanol solution) (equivalent to 12 equivalents to 18 equivalents of hydroxyl groups of α-CD molecules in the polyrotaxane). The suspension was stirred for 5 hours while distilling off the methanol under reduced pressure. 1.2 g of methyl iodide were added and after stirring for 19 hours, the reaction solution was diluted to 100 ml with purified water and the solution was dialyzed for 48 hours with a dialysis tube (fraction molecular weight: 12,000) in the presence of running tap water. Moreover, dialysis was repeated twice for 3 hours each in 500 ml of purified water followed by freeze-drying to obtain methylated polyrotaxane in which the OH groups of α-CD were substituted with OCH₃ groups. The yield was 0.97g.
¹H-NMR, (DMSO-d₆, 300MHz) δ (ppm) 3.0-4.0 (m, 1.8H), 4.43 (br, 1H), 4.75(br, m, 1H), 4.97(s, 1H), 5.4-5.8(br, 0.5H).

### Preparation Example 3: Hydroxypropylated Polyrotaxane

Cyclic molecules of the polyrotaxane of Preparation Example 1 were hydroxypropylated to obtain hydroxypropylated polyrotaxane of Preparation Example 3. The following provides a detailed description thereof.
5.0 g of the polyrotaxane of Preparation Example 1 were dissolved in 50 ml of 1N aqueous NaOH solution followed by the addition of 10 g of propylene oxide.
After stirring for 24 hours at room temperature, the solution was neutralized with hydrochloric acid. This solution was dialyzed for 48 hours with a dialysis tube (fraction molecular weight: 12,000) in the presence of running tap water. Moreover, dialysis was repeated four times for 12 hours each in 2000 ml of purified water. The dialyzed solution was then freeze-dried and the yield of the resulting product (hydroxypropylated polyrotaxane B-4) was 5.0 g (hydroxypropylation ratio: 33% with respect to OH groups).
¹H-NMR, (DMSO-d₆, 400MHz) δ (ppm) 1.0 (s, 3.0H), 3.1-4.0(m, 14.0H), 4.3-5.1(m, 3.1H), 5.3-6.0(m, 1.0H).

### Preparation Example 4: Methacryloylated Polyrotaxane

Methacryloyl groups were introduced into the hydroxypropylated polyrotaxane of Preparation Example 3 to obtain methacryloylated polyrotaxane of Preparation Example 4. The following provides a detailed description thereof.
0.5 g of the hydroxypropylated polyrotaxane of Preparation Example 3 were dissolved in 5 ml of 0.1 N NaOH followed by dropping 0.5 g of glycidyl methacrylate.
After stirring for 72 hours, the reaction liquid was neutralized with 1 N HCl aqueous solution followed by dialyzing the solution for 12 hours with a dialysis tube (fraction molecular weight: 12,000) in the presence of running tap water. Moreover, dialysis was repeated twice for 12 hours each in 2000 ml of purified water followed by freeze-drying to obtain methacryloylated polyrotaxane in which a portion of the OH groups were substituted with 3-methacryloyloxy-2-hydroxypropyl groups (introduction ratio: 0.4% with respect to hydroxyl groups). The yield was 0.5 g.
¹H-NMR, (DMSO-d₆, 400MHz) δ (ppm) 1.0 (s, 3.0H), 1.9(s, 0.04H) 3.0-4.1 (m, 13.7H), 4, 3-5.2(m, 3.0H), 5.3-6.2 (m, 0.9H) .

### Preparation Example 5: Hydroxypropylated Polyrotaxane

Polyrotaxane was prepared in the same manner as Preparation Example 1 with the exception of using a different PEG (number average molecular weight: 500,000) instead of the PEG used in Preparation Example 1. Cyclic molecules of the resulting polyrotaxane were hydroxypropylated in the same manner as Preparation Example 3 to prepare hydroxypropylated polyrotaxane (hydroxypropylation ratio: 27% with respect to hydroxyl groups) (yield: 45%, inclusion rate: 29%).

### Example 1 - Preparation of PEG-Containing Gel

450 mg of the polyrotaxane obtained in Preparation Example 1 were dissolved in 3 ml of dimethylsulfoxide (DMSO). 36 mg of carbonyl diimidazole (CDI) were added to this solution and allowed to react for 48 hours at 50°C to obtain crosslinked polyrotaxane. The resulting crosslinked polyrotaxane was placed in PEG (number average molecular weight: 300) to obtain a gel containing PEG in which the solvent had been substituted with PEG. In the case of assigning a value of 100% to the volume of the gel before PEG substitution, the volume of the gel after PEG substitution was 17% (degree of expansion: 1.2 times).

### Example 2 - Preparation of PEG-Containing Gel

450 mg of the methylated polyrotaxane obtained in Preparation Example 2 were dissolved in 3 ml of DMSO. 36 mg of CDI were added to this solution followed by allowing to react for 48 hours at 50°C to obtain crosslinked methylated polyrotaxane. The resulting crosslinked methylated polyrotaxane was immersed in an excess of PEG (number average molecular weight: 300) to obtain a gel containing PEG in which the solvent had been substituted with PEG. In the case of assigning a value of 100% to the volume of the gel before PEG substitution, the volume of the gel after PEG substitution was 60% (degree of expansion: 4.3 times).

### Example 3 - Preparation of PEG-Containing Gel

450 mg of the hydroxypropylated polyrotaxane obtained in Preparation Example 3 were dissolved in 3 ml of DMSO. 36 mg of CDI were added to this solution followed by allowing to react for 48 hours at 50°C to obtain crosslinked hydroxypropylated polyrotaxane. The resulting crosslinked hydroxypropylated polyrotaxane was immersed in an excess of PEG (number average molecular weight: 300) to obtain a gel containing PEG in which the solvent had been substituted with PEG. In the case of assigning a value of 100% to the volume of the gel before PEG substitution, the volume of the gel after PEG substitution was 67% (degree of expansion: 4.8 times).

### Example 4 - Preparation of PEG-Containing Gel

200 mg of the methylated polyrotaxane obtained in Preparation Example 2 were dissolved in 2 ml of a 0.03 N NaOH aqueous solution followed by the addition of 20 mg of divinylsulfone. This was then allowed to react for 48 hours at 5°C to obtain crosslinked methylated polyrotaxane. The resulting crosslinked methylated polyrotaxane was immersed in an excess of PEG (number average molecular weight: 300) to obtain a gel containing PEG in which the solvent had been substituted with PEG. In the case of assigning a value of 100% to the volume of the gel before PEG substitution, the volume of the gel after PEG substitution was 68% (degree of expansion: 6.9 times).

### Example 5 - Preparation of PEG-Containing Gel

200 mg of the hydroxypropylated polyrotaxane obtained in Preparation Example 3 were dissolved in 2 ml of a 0.03 N NaOH aqueous solution followed by the addition of 20 mg of divinylsulfone. This was allowed to react for 48 hours at 5°C to obtain crosslinked hydroxypropylated polyrotaxane. The resulting crosslinked hydroxypropylated polyrotaxane was immersed in an excess of PEG (number average molecular weight: 300) to obtain a gel containing PEG in which the solvent had been substituted with PEG. In the case of assigning a value of 100% to the volume of the gel before PEG substitution, the volume of the gel after PEG substitution was 71% (degree of expansion: 7.2 times).

### Example 6 - Preparation of PEG-Containing Gel

200 mg of the polyrotaxane obtained in Preparation Example 1 were dissolved in 2 ml of a 1 N NaOH aqueous solution followed by the addition of 20 mg of divinylsulfone. This was allowed to react for 48 hours at 5°C to obtain crosslinked polyrotaxane. The resulting crosslinked polyrotaxane was immersed in an excess of PEG (number average molecular weight: 300) to obtain a gel containing PEG in which the solvent had been substituted with PEG. In the case of assigning a value of 100% to the volume of the gel before PEG substitution, the volume of the gel after PEG substitution was 25% (degree of expansion: 2.5 times).

### Example 7 - Preparation of Gel Containing Polyethylene Glycol Dimethyl Ether

200 mg of the hydroxypropylated polyrotaxane obtained in Preparation Example 3 were dissolved in 2 ml of polyethylene glycol dimethyl ether (number average molecular weight: 250) followed by the addition of 0.05 ml of hexamethylene diisocyanate. The solution was gelled for 5 hours at 70°C to obtain a gel containing polyethylene glycol dimethyl ether (degree of expansion: 9.0 times).

### Example 8 - Photocrosslinking of Methacryloyl Group-Containing Polyrotaxane

100 mg of the polyrotaxane into which methacryloyl groups had been introduced obtained in Preparation Example 4 were dissolved in 1 ml of PEG (number average molecular weight: 300). 0.0014 g of 2-hydroxy-1-[4-(2-hydroxyethoxy)phenyl]-2- methyl-1-propanone were added thereto followed by stirring and irradiating with light using an ultra-high-pressure mercury lamp (350 W) to obtain a gel containing PEG (degree of expansion: 10.9 times).

### Comparative Example - Preparation of Hydrogel

3 g of the hydroxypropylated polyrotaxane obtained in Preparation Example 5 were dissolved in 20 ml of an 0.03 N NaOH aqueous solution followed by the addition of 0.2 g of divinylsulfone. This was reacted for 48 hours at 5°C to obtain crosslinked hydroxypropylated polyrotaxane. The resulting crosslinked hydroxypropylated polyrotaxane was allowed to stand for 1 day in pure water to obtain a hydrogel of crosslinked hydroxypropylated polyrotaxane.

### Example 9

Next, this hydrogel was immersed in PEG (number average molecular weight: 300) to obtain a gel in which the solvent was substituted with PEG. In the case of assigning a value of 100% to the volume of the gel before PEG substitution, the volume of the gel after PEG substitution was 73% (degree of expansion: 5.3 times).

### <Evaluation of Shock Absorbability>

The gel of Example 9 was formed to a thickness of 3 mm and cross-sectional area of 3 mm² followed by measurement of shock absorbability using a TMA/SS6100 thermomechanical analyzer (Seiko Instruments Inc.). In addition, the hydrogel prior to substitution with PEG in Example 9 was formed and measured in the same manner to serve as a comparative example. The vibration absorption coefficient (Tanδ) used as an indicator of shock absorbability at a frequency of 0.1 Hz was 0.1 for Example 9 and 0.01 for the comparative example.

### <Evaluation of Refractive Index>

The refractive index of the gel of Example 9 was evaluated using an Abbe refractometer (Atago Co., Ltd.). The hydrogel prior to substitution with PEG in Example 9 was measured in the same manner to serve as a comparative example. The refractive index at 20°C was 1.46 for Example 9 and 1.34 for the comparative example.

### <Evaluation of Moisture Permeability>

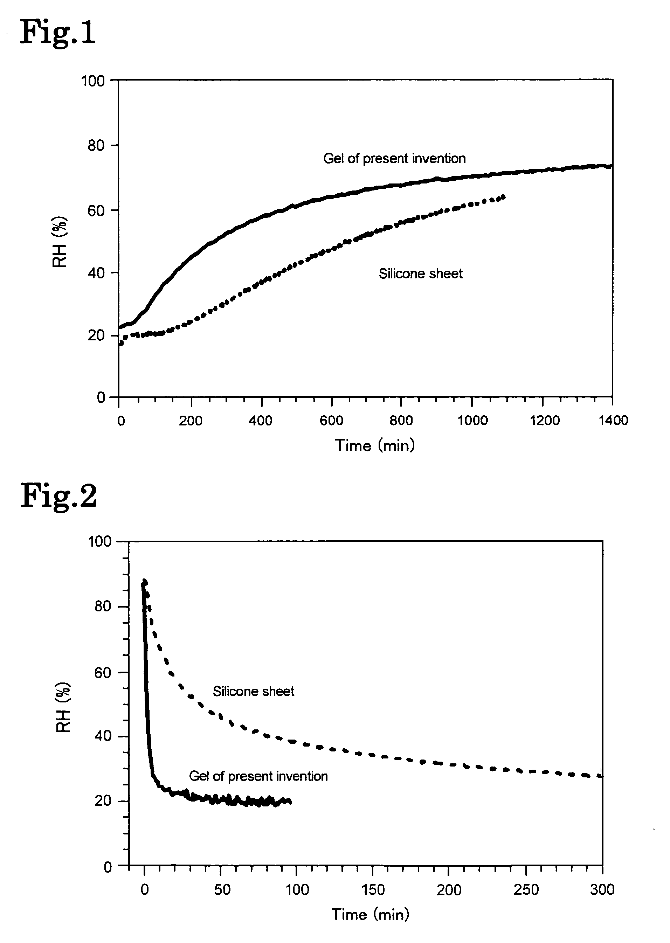

The gel of Example 9 was formed to a size of 84 x 84 x 2 mm followed by testing moisture permeability using a permeability tester with reference to JIS K7129. A silicone sheet (Azuwan Co., Ltd., catalog no. Al-1067-010-04) was formed and measured in the same manner for the sake of comparison. Those results are shown in Figs. 1 and 2. The sample was placed in the center of the sealed tester, the inside of the tester was divided into an upper cell and a lower cell, and the change in humidity over time in the upper cell was measured with a humidity sensor provided in the upper portion of the tester. Fig. 1 shows the change in humidity in the case of the upper cell being in a dry state and the lower cell being in a saturated state, while Fig. 2 shows the change in humidity in the case of the upper cell being in a saturated state and the lower cell being in a dry state. It can be understood from these graphs that the change in humidity for Example 9 is faster than that of a conventional silicone sheet, thereby making it superior particularly with respect to changes in humidity from a highly humid state.

### Example 10 - Photocrosslinking of Methacryloyl Group-Containing Polyrotaxane

75 mg of the polyrotaxane into which methacryloyl groups had been introduced obtained in Preparation Example 4 were mixed with 25 mg of polyethylene glycol diacrylate (number average molecular weight: 575, Aldrich) and 65 mg of PEG (number average molecular weight: 300). 0.5 mg of 2-hydroxy-1-[4-(2-hydroxyethoxy)phenyl]-2-methyl-1-propanone were added thereto followed by stirring and irradiating with light for 20 seconds using an ultra-high-pressure mercury lamp (350 W) to obtain a gel containing PEG. Measurement of the mechanical properties of this gel by preparing a test piece (strip, width: 4 mm, thickness: 0.5 mm, length: 10 mm) and using a RSA3 Rheometer (TA Instruments Ltd.) at a stretching speed of 0.2 mm/second yielded a Young's modulus of 0.55 kPa, maximum stress of 1.5 kPa and stretch rate of 200%.

### Example 11

### <Evaluation of Mechanical Properties: Effect of Photoirradiation Time>

A gel was prepared in the same manner as Example 8 and measurement of Young's modulus, maximum stress and stretch rate in the same manner as Example 10 resulted in the values shown for Example 11-1 in Table 1. Additional gels were also prepared while changing the photoirradiation time that yielded the results shown for Examples 11-2 and 11-3 in Table 1. There were no significant variations in values observed accompanying changes in photoirradiation time.

**Table 1**

| | Photoirradiation time (sec) | Young's modulus (kPa) | Maximum stress (kPa) | Stretch rate (%) |
|---|---|---|---|---|
| Example 11-1 | 5 | 7.4 | 5.9 | 64 |
| Example 11-2 | 20 | 6.8 | 5.6 | 73 |
| Example 11-3 | 60 | 7 | 5.7 | 68 |

### <Evaluation of Mechanical Properties: Effect of Amount of Polyrotaxane>

Gels were prepared in the same manner as Example 11-1 with the exception of using 200 mg or 300 mg of methacryloyl group containing polyrotaxane. Measurement of Young's modulus, maximum stress and stretch rate in the same manner as Example 10 resulted in the values shown for Examples 11-4 and 11-5 in Table 2. Young's modulus, maximum stress and stretch rate all increased when the amount of polyrotaxane was increased.

**Table 2**

| | Methacryloyl group containing polyrotaxane (mg) | Irradiation time (sec) | Young's modulus (kPa) | Maximum stress (kPa) | Stretch rate (%) |
|---|---|---|---|---|---|
| Example 11-1 | 100 | 5 | 7.4 | 5.9 | 64 |
| Example 11-4 | 200 | 5 | 54.1 | 54 | 91 |
| Example 11-5 | 300 | 5 | 147 | 173 | 153 |

According to the results for the examples as indicated above, the gel composition of the present invention was determined to demonstrate improved shock absorbability.
In addition, use of the gel composition of the present invention was determined to be able to increase refractive index. Moreover, moisture permeability was determined to be obtained that is superior to silicone sheets conventionally used as materials having superior moisture permeability.

### INDUSTRIAL APPLICABILITY

Since the gel composition of the present invention uses a material having a network structure containing a polyrotaxane, in addition to being able to expect that various properties attributable to polyrotaxane will be retained, stability is easily ensured as a result of containing a non-aqueous solvent, thereby enabling this gel composition to be applied to various products. Examples of such products include, rubber bands, packing materials, agar media, fabrics, cushioning materials for soles of shoes such as sports shoes, shock-absorbing materials (bumpers) for automobiles and various devices, toys utilizing high water absorption, coatings for rubbing portions of devices (for example, coatings for housings or sliding parts of pumps), adhesives, sealing materials for sealing, dehumidifiers or condensed moisture removers utilizing water-absorbing property, fillers for bed (like a waterbed) mats, materials for special effects or models, soft contact lens materials (especially soft content lens materials having a high water content and/or superior strength), tire materials, electrophoretic gels, new foodstuffs corresponding to gum and other products, gum for dogs, biomaterials such as artificial corneas, lenses, vitreous bodies, skin, muscle, joints or cartilages, including biocompatible materials such as breast implant materials, medical materials for external application such as wet compress materials or wound dressings, drug delivery systems, earplugs, wet suits, protective mats installed on outfield fences in baseball stadiums, arm rests for personal computers, disposable sanitary articles such as children's diapers, sanitary napkins or adult diapers, photographic photosensitive materials, aromatics, coating agents such as coatings, including various paints and the aforementioned coatings, functional membranes for separation, water-swellable rubber, water-resistant tape, gabions or sandbags, materials for pile extraction, materials for removing water in oil, moisture conditioning materials, hydroscopic gelling agents, dehumidifiers, materials for artificial snow in indoor artificial ski slopes, refractory coatings for buildings, landslide prevention materials, concrete products such as concrete-laying materials, sludge gelling agents, agents for preventing sludge leakage, tree-planting materials such as water-in-soil retaining agents or seedling media, materials for chromatographic carriers, materials for bioreactor carriers, and various composite materials for fuel cells such as various types of cell materials including electrolytes.

In particular, the gel composition of the present invention has superior shock absorbability, making it suitable for sporting goods such as sports shoes and rackets, construction materials such as vibration isolating and vibration dampening materials, and medical materials such as supporters and liners for artificial legs.

In addition, since the gel composition of the present invention facilitates control of refractive index, it can be used as an alternative material to clear plastics (such as PMMA). It is particularly suitable for soft contact lens materials, artificial corneas, artificial lenses, artificial vitreous bodies and other ophthalmic devices.

Moreover, since the gel composition of the present invention has superior moisture permeability in the case the medium contains polyethylene glycol in particular, it is suitable for medical materials such as supporters and liners of artificial legs.

## Claims

1. A gel composition comprising a material having a network structure containing a polyrotaxane and a non-aqueous solvent.

2. The gel composition according to claim 1, wherein the network structure containing a polyrotaxane is a structure in which polyrotaxanes are crosslinked each other.

3. The gel composition according to claim 2, wherein the structure in which polyrotaxanes are crosslinked each other is a structure in which cyclic molecules of polyrotaxanes are crosslinked each other by physical bonds and/or chemical bonds.

4. The gel composition according to claim 1, wherein the network structure containing a polyrotaxane is a structure in which a polyrotaxane and a polymer are crosslinked.

5. The gel composition according to claim 4, wherein the structure in which a polyrotaxane and a polymer are crosslinked is a structure in which cyclic molecules of polyrotaxane and a polymer are crosslinked by physical bonds and/or chemical bonds.

6. The gel composition according to any one of claims 1 to 5, wherein the non-aqueous solvent is a natural oil, polyvalent alcohol, fatty acid, ether, ethyl abietate or silicone oil.

7. The gel composition according to claim 6, wherein the non-aqueous solvent is a polyethylene glycol having a number average molecular weight of 200 to 600.

8. The gel composition according to any one of claims 1 to 7, wherein a weight of the gel composition to a dry weight of the material having a network structure containing a polyrotaxane is 1.1 to 1000.

9. A process for preparing a gel composition comprising a material having a network structure containing a polyrotaxane and a non-aqueous solvent, comprising:
1) crosslinking at least a portion of cyclic molecules of polyrotaxanes with each other by physical bonds and/or chemical bonds; and,
2) immersing in a medium containing the non-aqueous solvent.

10. A process for preparing a gel composition comprising a material having a network structure containing a polyrotaxane and a non-aqueous solvent, comprising:
crosslinking at least a portion of cyclic molecules of polyrotaxanes with each other by physical bonds and/or chemical bonds in a medium containing the non-aqueous solvent.

11. A process for preparing a gel composition comprising a material having a network structure containing a polyrotaxane and a non-aqueous solvent, comprising:
1) mixing a polyrotaxane and a polymer;
2) crosslinking at least a portion of the polymer with at least a portion of cyclic molecules of the polyrotaxane, and optionally, at least a portion of the polymer with each other, by physical bonds and/or chemical bonds; and
3) immersing in a medium containing the non-aqueous solvent.

12. A process for preparing a gel composition comprising a material having a network structure containing a polyrotaxane and a non-aqueous solvent, comprising:
1) mixing a polyrotaxane and a polymer;
2) crosslinking at least a portion of the polymer with at least a portion of cyclic molecules of the polyrotaxane, and optionally, at least a portion of the polymer with each other, by physical bonds and/or chemical bonds in a medium containing the non-aqueous solvent.

13. The process for preparing a gel composition according to any one of claims 9 to 12, wherein the non-aqueous solvent is a natural oil, polyvalent alcohol, fatty acid, ether, ethyl abietate or silicone oil.

14. The process for preparing a gel composition according to claim 13, wherein the non-aqueous solvent is a polyethylene glycol having a number average molecular weight of 200 to 600.

15. A gel composition obtained according to the process according to any one of claims 9 to 14.

16. A sporting good, construction material or medical material containing the gel composition according to any one of claims 1 to 8 and 15.

17. An ophthalmic device containing the gel composition according to any one of claims 1 to 8 and 15.
